Europäisches Patentamt

(19) European Patent Office

Office européen des brevets

(11) Publication number: **0 137 364**
A2

# (12) EUROPEAN PATENT APPLICATION

(21) Application number: **84111104.0**

(22) Date of filing: **18.09.84**

(51) Int. Cl.⁴: **A 61 K 9/02**

(30) Priority: **23.09.83 US 535098**

(43) Date of publication of application:
**17.04.85 Bulletin 85/16**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065(US)**

(72) Inventor: **Allegretti, John E.**
**31 Hamlin Road**
**East Brunswick New Jersey 08816(US)**

(74) Representative: **Abitz, Walter, Dr.-Ing. et al,**
**Abitz, Morf, Gritschneder, Freiherr von Wittgenstein**
**Postfach 86 01 09**
**D-8000 München 86(DE)**

(54) Suppository form of an osmotic therapeutic system.

(57) A suppository of usual size, shape and materials of construction is disclosed which is well suited for its intended purpose, incorporating therein an art-recognized osmotic therapeutic system for delivery of a therapeutic agent, such as indomethacin, via the anal canal.

EP 0 137 364 A2

- 1 -                                17002

TITLE OF THE INVENTION

SUPPOSITORY FORM OF AN OSMOTIC THERAPEUTIC SYSTEM

BACKGROUND OF THE INVENTION

Osmotic therapeutic systems are known from U.S. Patent 4,256,108 and 4,265,874 and are described therein for administration via most or all body orifices including the rectum. However, these devices employing a usual dose of any medicament, and certainly indomethacin, are quite small being about the size of an aspirin tablet and are dry walled making for difficult and uncomfortable administration.

Now with the present invention there is provided an easily administrable suppository comprising an osmotic therapeutic device of normal size and shape with the combined attendant advantages of the osmotic device and of rectal administration.

DETAILED DESCRIPTION OF THE INVENTION

The novel product of this invention is a suppository comprised of a suppository base which melts or dissolves in the rectal fluids having incorporated therein an osmotic therapeutic system as described in U.S. Patents 4,256,108 and 4,265,874.

In particular the novel product of this invention is a suppository incorporating therein an osmotic therapeutic system as described in U.S. Patents 4,256,108 and 4,265,874 comprising indomethacin as active ingredient.

The osmotic device incorporated in the suppository of this invention houses from 40 to 250 mg of sodium indomethacin and more preferably from 85 to 125 mg of sodium indomethacin trihydrate the equivalent of 70 to 100 mg of indomethacin, from 50 to 300 mg of potassium bicarbonate and more preferably from 130 to 190 mg of potassium bicarbonate, 2 to 25 mg of excipients, and more preferably 5 to 20 mg. The device has an inner microporous forming lamina weighing 18 to 25 mg with a thickness of 0.075 to 0.175 mm, and an exterior semipermeable lamina weighing 6 to 20 mg with a thickness of 0.05 to 0.15 mm. The device has a passageway of 0.254 to 0.381 mm, and releases drug at the rate of 2 to 15 mg/hr.

EXAMPLE 1

An osmotic device for the delivery of a nonsteroidal anti-inflammatory drug sodium indomethacin, was manufactured as follows: a drug composition was prepared for housing in the compartment of the device by thoroughly blending 105.4 mg of sodium indomethacin trihydrate, 158 mg of potassium bicarbonate, 8.4 mg of povidone USP, and 8.2 mg of stearic acid, and then compressing the homogeneous blend into a precompartment forming drug formulation. Next, the compressed drug formulation was placed in an air suspension equipment and coated with a microporous lamina forming composition. The microporous lamina composition comprised 45% by weight of cellulose acetate having an acetyl content of 39.8%, 45% by weight of sorbitol, and 10% by weight of polyethylene glycol 400. The lamina was formed from a methylene chloride - 95% ethanol solvent (80:20 wt:wt). The microporous lamina was 0.127 mm thick.

Next, an exterior semipermeable lamina was laminated onto the microporous lamina, in the air suspension machine. The semipermeable lamina forming composition comprised 90% by weight of cellulose acetate having an acetyl content of 39.8% and 10% by weight of cellulose acetate having an acetyl content of 32%. The semipermeable lamina was applied from a solvent mixture comprising methylene chloride and 95% ethanol, 80:20 wt:wt. The systems were dried, and a 0.254 to 0.381 mm passageway was laser drilled through the laminated wall. The system releases indomethacin at the rate of 4 mg per hour. The device in operation, releases a solution that effervesces on contact with the colonic fluid at the exit end of the passageway, producing carbon dioxide bubbles that disperse the drug in a fluffy state.

## EXAMPLE 2

An osmotic device for the controlled and continuous delivery of indomethacin was made by following the general procedure described above. In the present device, the compartment housed a drug formulation comprising 56.4% potassium bicarbonate, 37.6% sodium indomethacin trihydrate, 3% povidone, USP and 2.9% stearic acid. The formulation after compressing had a diameter of 7.93 mm, an area of 1.6 $cm^2$ and a density of 1.65 g/ml. The device had a laminated wall comprising an interior microporus lamina consisting essentially of 45% by weight of cellulose acetate having an acetyl content of 39.8%, 45% by weight of sorbitol, and 10% by weight of polyethylene glycol 400. The lamina was applied from a solvent comprising methylene chloride-methanol-water. 62:35:3 by wt. A semipermeable lamina was laminated onto the microporous lamina, which semipermeable lamina consists of 50% by weight of cellulose acetate having an acetyl content of 39.8%, and 50% by weight of cellulose acetate having an acetyl content of 32%. The lamina was applied from a solvent consisting of methylene chloride and methanol, 80:20 by wt. The microporous forming lamina was 0.127 mm thick, and the semipermeable lamina 0.061 mm thick. The device had a 0.254 to 0.381 mm passageway and delivered indomethacin at the rate of 7 mg/hr. The device delivers the drug substantially free of rapid precipitation at the passageway environment interface, and on the wall of the device in the vicinity of the passageway.

0137364

## EXAMPLE 3

The procedure of Example 2 was repeated with the conditions as described except that the microporous forming lamina was 0.0254 mm thick, the semipermeable lamina 0.0686 mm thick, and the rate of release was 8 mg/hr.

## EXAMPLE 4

The osmotic devices of Examples 1 and 2 were manufactured in this example, wherein (a) the microporous lamina was 0.127 mm thick, the semipermeable lamina was 0.0864 mm thick, and the device had a release rate of 6 mg/hr. and (b) a device wherein the microporous lamina was 0.127 mm thick, the semipermeable lamina was 0.0432 mm thick, and the system had a release rate of 12 mg/hr.

## EXAMPLE 5
### Suppository Formulations

| | | |
|---|---|---|
| A. | Osmotic device | 1 |
| | Suppocire L qs ad | 3 g. |
| | | |
| B. | Osmotic device | 1 |
| | Polyethylene glycol 1540 | 3.006 g. |
| | Polyethylene glcyol 6000 | 0.344 g. |

In each of formulations A and B one of the devices described in Examples 1 through 4 is incorporated in the suppository base by standard procedures.

## WHAT IS CLAIMED IS:

1. A suppository comprised of a suppository base which melts or dissolves in the rectal fluids, having incorporated therein an osmotic therapeutic system.

2. The suppository of Claim 1 wherein the osmotic therapeutic system incorporates indomethacin as the active ingredient.

3. The suppository of Claim 2 wherein the osmotic therapeutic system houses from 40 to 250 mg of sodium indomethacin trihydrate, 50 to 300 mg of potassium bicarbonate, 2 to 25 mg of excipients and is comprised of an inner microporous forming lamina weighing 18 to 25 mg with a thickness of 0.10 to 0.16 mm and an exterior semipermeable lamina weighing 6 to 20 mg with a thickness of 0.035 to 0.100 mm, having a passageway of 0.254 to 0.381 mm diameter whereby drug is released at a rate of 2 to 15 mg/hr.

4. The suppository of Claim 3 wherein the osmotic therapeutic system houses 85 to 125 mg of sodium indomethacin trihydrate, 130 to 190 mg of potassium bicarbonate and 5 to 20 mg of excipients.